# FASCICULE DE BREVET EUROPEEN

(11) **EP 1 494 627 B1**
(45) Date de publication et mention de la délivrance du brevet: **11.06.2008**
(21) Numéro de dépôt: 03746347.8
(22) Date de dépôt: 15.04.2003
(51) Int. Cl.: A61F 5/01, B29C 39/00

(54) **ATTELLE POUR ARTICULATION ET PROCEDES DE FABRICATION D'UNE TELLE ATTELLE**
SCHIENE FÜR EINE GELENKVERBINDUNG UND VERFAHREN ZUR HERSTELLUNG EINER SOLCHEN SCHIENE
SPLINT FOR A JOINT CONNECTION AND METHODS FOR PRODUCTION OF SUCH A SPLINT

(30) Priorité: 15.04.2002 FR 0205352
(43) Date de publication de la demande: 12.01.2005
(73) Titulaire: Parizot, Jean-Paul, 21000 Dijon (FR)
(72) Inventeur: Parizot, Jean-Paul, 21000 Dijon (FR)
(74) Mandataire: Callon de Lamarck, Jean-Robert
(86) Numéro de dépôt international: PCT/FR2003/001207
(87) Numéro de publication internationale: WO 2003/086248

(56) Documents cités:
- FR-A- 2 721 037
- US-A- 4 661 535
- US-A- 5 007 416
- US-A- 5 027 801
- US-A- 5 445 602
- US-A- 5 695 452
- US-A- 5 713 837
- US-A- 5 716 335

## Description

La présente invention concerne une attelle pour une articulation reliant deux membres d'un corps humain ou d'un animal, telle que la cheville, le genou ou le coude par exemple, constituée d'au moins deux coques rigides globalement concaves, aptes à être positionnées de part et d'autre de l'articulation, en appui sur ladite articulation, comprenant des moyens permettant d'éviter toute blessure du tissu oedémateux apparu consécutivement à une entorse ou une foulure de ladite articulation.

Dans le domaine de la traumatologie notamment sportive, on connaît bien des orthèses pour chevilles communément appelées attelles permettant d'éviter les mouvements d'éversion et d'inversion du pied tout en permettant une flexion normale de ce dernier soit en prévention d'une entorse ou d'une foulure de la cheville, lors de la pratique d'un sport par exemple, soit de manière à favoriser la résorption de l'oedème résultant d'une entorse et situé autour de la cheville. Ces attelles sont habituellement constituées de deux coques rigides globalement concaves, aptes à être positionnées de part et d'autre de la cheville, en appui sur ladite cheville, et comprenant respectivement sur leurs faces internes, c'est-à-dire sur leurs faces concaves, une chambre réalisée en une matière plastique souple pouvant être mise sous pression par tout moyen approprié. Ces chambres sont positionnées sur la face interne de chaque coque pour fournir un coussin de support entre chaque coque et la cheville, et elles recouvrent une partie au moins de la face interne de chaque coque de telle sorte que, à chaque pas, ces chambres exercent une compression sur les tissus oedémateux procurant un effet de massage qui contribue à la disparition rapide des oedèmes. Par ailleurs, l'attelle comprend des moyens pour maintenir lesdites coques en position de part et d'autre de l'articulation constitués de bande de tissus entourant lesdites coques de l'attelle.

Une telle attelle est décrite dans le brevet européen EP 0 252 121 déposé par la société AIRCAST concernant une attelle de cheville. Cette attelle est constituée d'une enveloppe extérieure comprenant deux coques rigides concaves aptes à être positionnées de part et d'autre de la cheville et une base également rigide apte à être positionnée sous le talon. La base comprend deux pattes obtenues dans une bande de tissus s'étendant de part et d'autre de ladite base et comprenant sur leurs faces externes, à leurs extrémités libres respectives, des boucles aptes à coopérer avec des crochets du type « velcro », qui est une marque déposée, moulés dans la paroi interne de chaque coque juste au dessus d'une lumière horizontale globalement rectangulaire située à l'extrémité inférieure de chaque coque, chaque patte étant enfilée dans la lumière de sa coque respective depuis l'extérieur vers l'intérieur de la coque. L'attelle comprend, par ailleurs, une première et une seconde chambres pouvant être mises sous pression pour fournir un coussin de support entre chaque coque et la cheville, la seconde chambre s'étendant le long de la partie inférieure de la première chambre à proximité de la base. De plus, l'attelle comprend des moyens pour maintenir les coques en contact avec la jambe de telle sorte que la pression appliquée contre la cheville par la seconde chambre soit relativement plus importante que la pression appliquée contre la jambe par la partie de la première chambre s'étendant au dessus de la seconde chambre.

Ce type d'attelle présente l'inconvénient d'avoir des bords rigides susceptibles de prendre appui sur le tissu oedémateux de la cheville occasionnant une gêne et des douleurs procurées par la pression qu'exerce les bords rigides des coques. Par ailleurs, les pattes de la base de l'attelle étant solidarisées sur les parois internes des coques, il est nécessaire de retirer entièrement l'attelle pour pouvoir accéder auxdites pattes et régler de manière adéquate la hauteur des coques par rapport à la base, de sorte que l'attelle est dans la plupart des cas mal positionnée, ce qui gêne considérablement les utilisateurs de ces attelles.

Afin de remédier à ces inconvénients, on a déjà imaginé des attelles constituées d'une copie rigide centrale munie le long de ses bords longitudinaux de garnitures obtenues dans un matériau résilient ; c'est le cas, par exemple du brevet américain US 5 716 335. Ces garnitures sont obtenues dans du caoutchouc ou similaire et sont collées sur les bords longitudinaux de la coque rigide qui comprend des rainures pour permettre le couplage mécanique des garnitures avec la coque rigide centrale obtenue dans du polyéthylène haute densité (HDPE), du nylon ou du nylon chargé de verre commercialisé par la Société DUPONT (marque déposée).

Les garnitures de ces attelles présentent l'inconvénient de se décoller, voire même de s'arracher en cas d'utilisation prolongée et répétée de ces attelles, les rendant ainsi totalement inefficaces. De plus, les frottements du caoutchouc sur la peau d'un patient portant l'attelle pied nu sont particulièrement désagréables et risquent de provoquer des brûlures.

L'un des buts de l'invention est donc de remédier à cet inconvénient en proposant une attelle pour une articulation reliant deux membres d'un corps humain ou d'un animal, telle que la cheville par exemple, de conception simple et permettant d'éviter toute blessure du tissu oedémateux apparu consécutivement à une entorse ou une foulure de ladite articulation.

A cet effet, et conformément à l'invention, il est proposé une attelle pour une articulation reliant deux membres d'un corps humain ou d'un animal, telle que la cheville, le genou ou le coude par exemple, constituée d'au moins une coque rigide globalement concave, constituée d'un unique élément comprenant au moins une zone flexible, apte à être positionnée autour de l'articulation, en appui sur ladite articulation, et comprenant respectivement sur la face interne, c'est-à-dire sur la face concave, une chambre réalisée en une matière plastique souple pouvant être mise sous pression par tout moyen approprié, positionnée sur la face interne de la coque pour fournir un coussin de support entre ladite coque et l'articulation, et recouvrant une partie au moins de la face interne de la coque, ladite attelle comprenant des moyens pour maintenir ladite coque en position autour de l'articulation ; cette attelle est remarquable en ce que la zone flexible est obtenue dans un co-polymère blocs styrène éthylène butylène styrène (SEBS) chimiquement lié à la partie rigide de la coque afin d'éviter toute blessure du tissu oedémateux apparu consécutivement à une entorse ou une foulure de ladite articulation.

Selon une caractéristique essentielle de l'attelle conforme à l'invention, la coque est rigide dans sa partie centrale et flexible le long de ses bords longitudinaux.

On comprend bien que, les bords des coques étant flexibles, ces derniers exercent une pression réduite sur le tissu oedémateux résultant d'une entorse ou d'une foulure lors de la flexion du pied favorisant ainsi une résorption rapide de l'oedème. De plus, outre le fait que le SEBS qui est un matériau agréable au toucher améliore la sensation de confort pour un utilisateur portant l'attelle pied nu, la coque étant obtenue en une seule partie, c'est-à-dire issue d'un unique moule, la zone flexible de la coque ne s'arrache pas contrairement aux dispositifs de l'art antérieur.

Un autre objet de l'invention concerne un procédé de fabrication de la ou des coques d'une attelle pour une articulation reliant deux membres d'un corps humain ou analogue, telle que la cheville, le genou ou le coude par exemple, constituée d'au moins une coque rigide globalement concave, apte à être positionnée autour de l'articulation, en appui sur ladite articulation.

Ce procédé consiste à introduire une matière synthétique liquide à chaud qui se rigidifie en refroidissant dans un moule de forme correspondant à la forme de la coque à obtenir, puis à introduire dans ledit moule une matière souple dans au moins une zone dudit moule.

Selon une variante d'exécution du procédé conforme à l'invention, la matière souple est introduite dans la matière synthétique.

D'autres avantages et caractéristiques ressortiront mieux de la description qui va suivre, donnée à titre d'exemple non limitatif, d'une attelle conforme à l'invention en référence aux dessins annexés sur lesquels :
- la figure 1 est une vue en perspective éclatée d'une attelle pour cheville de pied gauche conforme à l'invention,
- la figure 2 est une vue de côté de la coque externe de l'attelle pour cheville conforme à l'invention représentée sur la figure 1,
- la figure 3 est une vue de côté de la coque interne de l'attelle pour cheville conforme à l'invention représentée sur la figure 1,
- la figure 4 est une vue en coupe frontale de l'attelle pour cheville positionnée sur la cheville d'un individu,
- la figure 5 est une vue en plan d'une bande formant les moyens pour maintenir les coques de l'attelle suivant l'invention en position en position de part et d'autre de la cheville,
- la figure 6 est une vue de côté de la bande représentée sur la figure 5,
- la figure 7 est une vue en perspective éclatée d'une chambre pouvant être mise sous pression afin de former un coussin de support entre chaque coque et la cheville,
- la figure 8 est une vue de dessus de la base de l'attelle pour cheville conforme à l'invention
- la figure 9 est une vue en coupe suivant l'axe IX-IX' d'une variante d'exécution de la coque externe de l'attelle conforme à l'invention représentée sur la figure 2
- la figure 10 est une vue en coupe suivant l'axe X-X' de la variante d'exécution de la coque de l'attelle conforme à l'invention représentée sur la figure 9.

On décrira, dans cet exemple non limitatif, une attelle pour cheville conforme à l'invention adaptée pour la cheville gauche d'un individu.

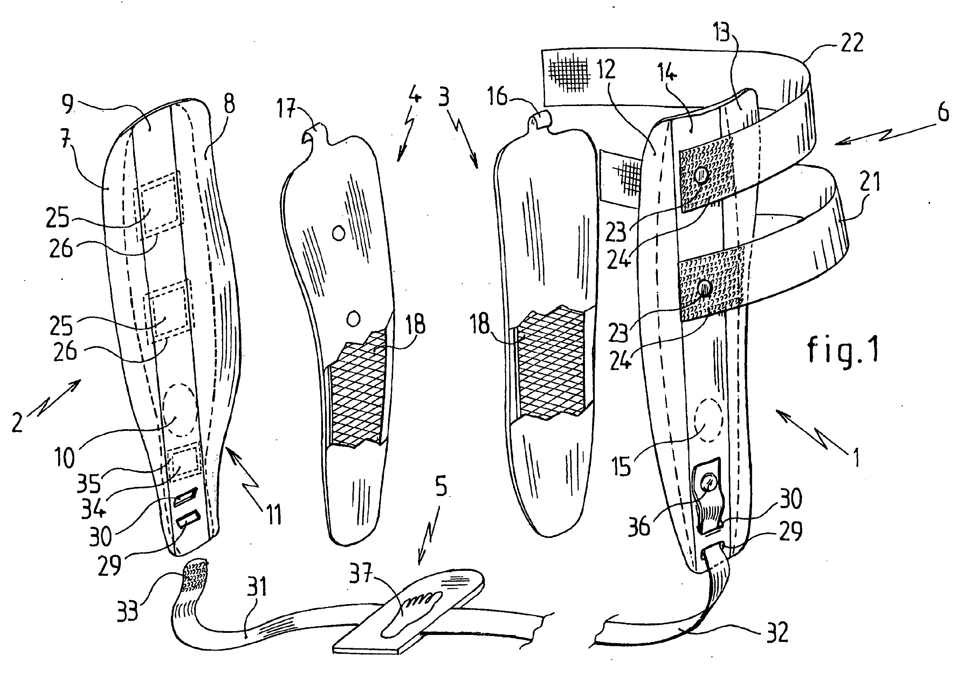

L'attelle pour cheville, représentée verticalement sur la figure 1, est constituée de deux coques rigides globalement concaves, une première coque dite interne 1 apte à être positionnée du côté interne de la cheville et une seconde coque dite externe 2 apte à être positionnée du côté externe de ladite cheville.

Chaque coque interne et externe 1 et 2 comprend sur leurs parois internes respectives, c'est-à-dire leurs parois concaves qui sont en regard de la cheville lorsque l'attelle est positionnée autour de cette dernière, une chambre respectivement 3 et 4 réalisée en une matière plastique souple pouvant être mise sous pression par tout moyen approprié, tel qu'une pipette par exemple, positionnée sur la paroi interne de chaque coque 1 et 2 pour fournir un coussin de support entre chaque coque et la cheville. L'attelle comprend, par ailleurs, une base 5 sur laquelle prend appui le talon du pied et des moyens 6 pour maintenir lesdites coques 1 et 2 en position de part et d'autre de la cheville, la base 5 et les moyens 6 seront décrits avec plus de précision un peu plus loin.

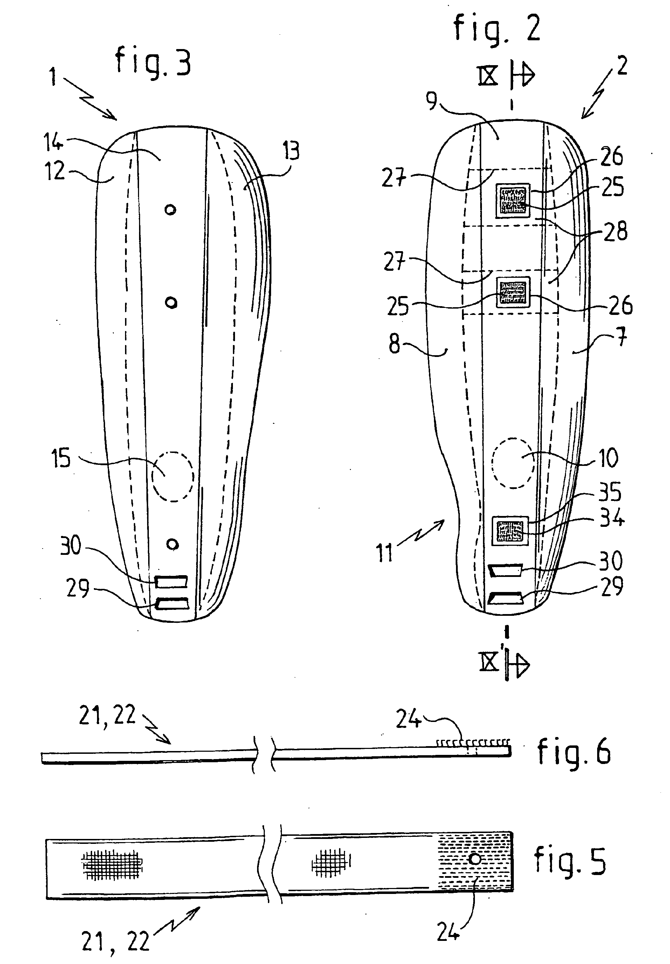

En référence aux figures 1 et 2, la coque externe 2 comprend le long de ses bords longitudinaux respectivement une zone flexible 7 et 8 afin d'éviter toute blessure du tissu oedémateux apparu consécutivement à une entorse ou une foulure de la cheville, les zones flexibles 7 et 8 exerçant une pression réduite sur le tissu oedémateux lors de la flexion du pied ce qui favorise une résorption rapide de l'oedème. On notera que la partie centrale 9 de la coque externe 2 s'étendant depuis son extrémité inférieure jusqu'à son extrémité supérieure est rigide ; à cet égard, l'épaisseur de la coque externe dans sa partie centrale est de préférence supérieure à l'épaisseur des zones flexibles 7 et 8 de ladite coque externe 2. Accessoirement, la coque externe 2 comprend dans sa partie centrale une zone flexible 10 globalement circulaire correspondant à une zone d'appui de ladite coque externe sur la malléole externe de la cheville qui forme une excroissance de cette articulation. Les zones flexibles 7, 8 ou 10 consistent dans une zone de matière souple, du SEBS, c'est-à-dire du co-polymère blocs styrène éthylène butylène styrène (élastomère thermoplastique), mélangé avec de la matière synthétique rigide tel que du polypropylène copolymère (PPc) ou du polyamide 6 (PA 6), dans laquelle est obtenue la partie rigide de la coque externe 2.

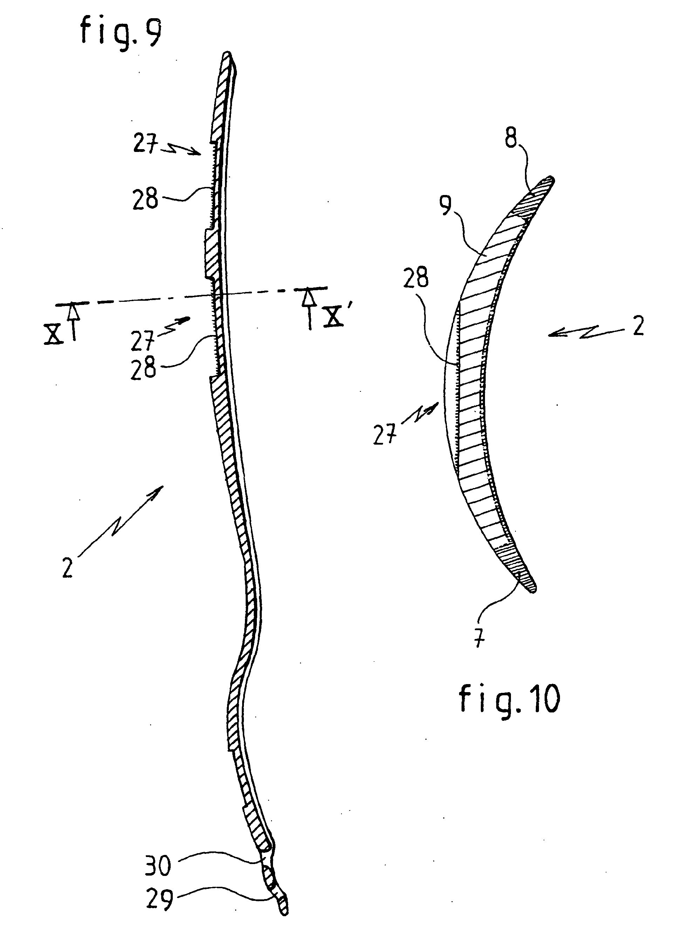

Selon une variante d'exécution de l'attelle pour cheville suivant l'invention, en référence à la figure 10, les zones flexibles 7, 8 ou 10 sont obtenues en introduisant une matière synthétique, telle que du PPc ou du PA6 par exemple, liquide à chaud qui se rigidifie en refroidissant dans un moule de forme correspondant à la forme de la coque afin d'obtenir la partie centrale rigide 9, puis en introduisant dans ledit moule une matière souple, du SEBS, dans la zone du moule correspondant à paroi interne de la coque de sorte que la matière souple s'étende de part et d'autre de la partie centrale rigide 9 pour former les zones flexibles 7 et 8 et sur la paroi interne de ladite coque. On notera que le SEBS qui est un matériau particulièrement agréable au toucher améliore la sensation de confort pour un utilisateur pied nu par exemple.

Par ailleurs, en référence aux figures 1 et 2, la coque externe 2 comprend le long de son bord latéral avant, c'est-à-dire le bord latéral vertical situé à droite des figures 1 et 2, à proximité de son extrémité inférieure, une échancrure 11 afin d'éviter toute pression du bord latéral contre la bosse du coup de pied lors d'une flexion de la jambe.

En référence aux figures 1 et 3, la coque interne 1 comprend, de la même manière que précédemment, le long de ses bords longitudinaux, respectivement une zone flexible 12 et 13 délimitée par des traits pointillés. La coque interne est rigide dans sa partie centrale 14 et présente, à cet égard, une épaisseur plus grande que l'épaisseur des zones flexibles. Accessoirement, la coque interne 1 comprend dans sa partie centrale rigide 14 une zone flexible 15 correspondant à la zone d'appui de la coque interne 1 sur la malléole interne de la cheville.

Les chambres 3 et 4, en référence aux figures 1, 4 et 7 sont solidarisées aux parois internes des coques respectivement interne 1 et externe 2 par tout moyen approprié telle que de la colle ou des moyens de fixation du type Velcro (marque déposée) de telle sorte qu'elle recouvre la paroi interne de chacune des coques 1 et 2 afin de fournir un coussin de support entre chaque coque 1 et 2 et la cheville. Les chambres 3 et 4 présentent une forme globalement triangulaire et comprennent une valve 16 et respectivement 17 apte à coopérer avec tout moyen approprié, telle qu'une pipette consistant dans un tube souple, afin de mettre sous pression lesdites chambres 3 et 4, la mise sous pression s'effectuant en soufflant dans la pipette introduite dans la valve 16 ou 17 de la chambre 3 ou 4. De manière particulièrement avantageuse, chaque chambre 3 et 4 comprend un élément compressible poreux 18 positionné à l'intérieur de chacune des chambres 3 et 4 remplissant sensiblement le volume intérieur de chacune desdites chambres 3 et 4 lorsque ces dernières ne sont pas mises sous pression. L'élément compressible poreux 18 consiste de préférence dans une mousse à résilience lente telle que la mousse polyether à basse résistance 1.50 LR3 commercialisée par la Société TRAMICO par exemple. On entend par mousse à résilience lente une mousse qui se déforme sous l'effet d'une pression et qui revient très lentement dans sa position initiale.

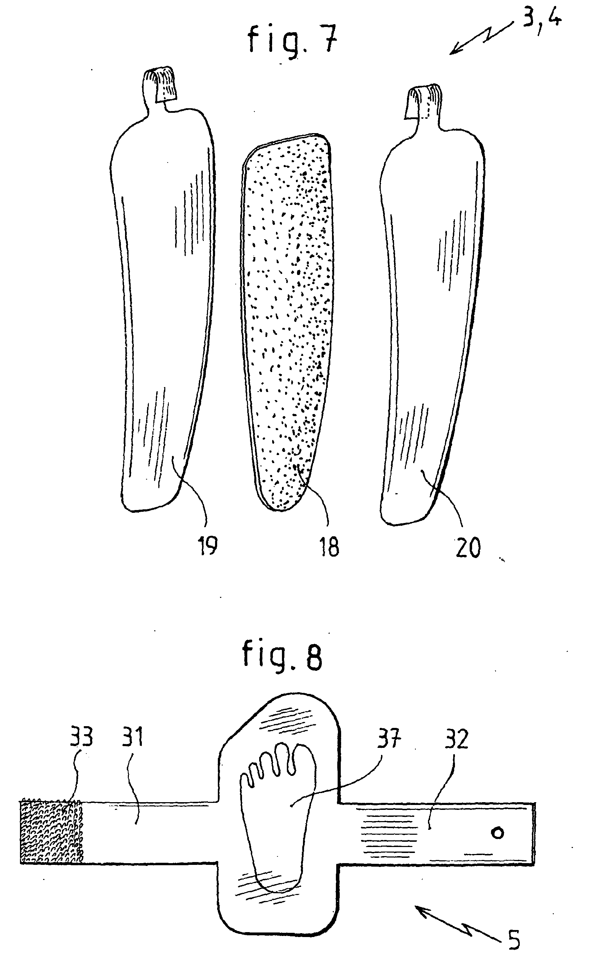

En référence à la figure 7, les chambres 3 et 4 sont obtenues en thermosoudant deux feuilles 19 et 20 globalement triangulaires réalisées en une matière plastique souple telle que l'éthyle vinyle acétylène (E.V.A.), et de préférence du polyuréthane, thermo-soudées le long de leurs bords, l'élément compressible poreux 18 étant positionné entre les deux feuilles 19 et 20 avant leur thermo-soudage.

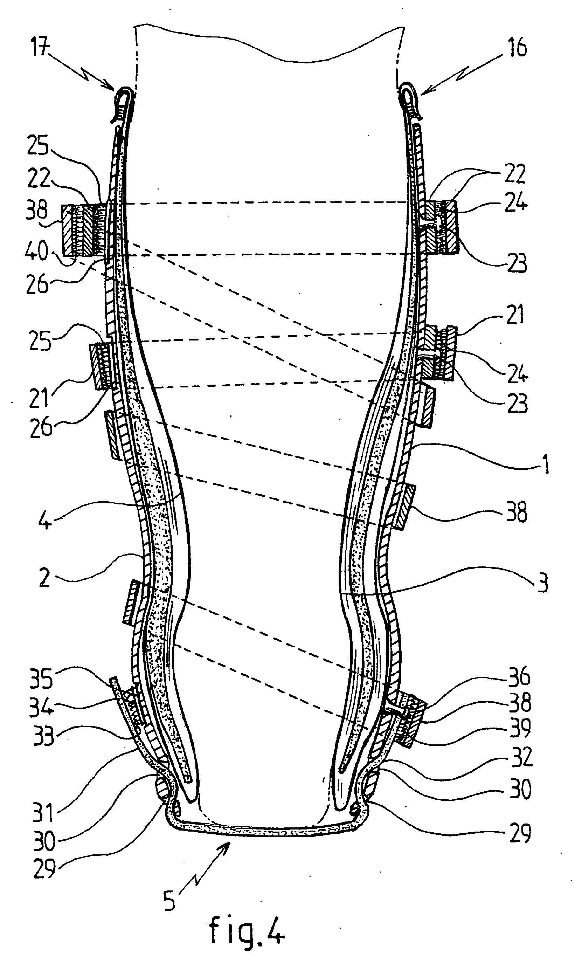

En référence aux figures 1 et 4, les moyens 6 pour maintenir en position les coques interne 1 et externe 2 de part et d'autre de la cheville sont constituées de deux bandes de tissu 21, 22 velouté dont une extrémité libre est respectivement solidarisée à la paroi externe de la coque interne 1 de l'attelle par un moyen de fixation tel qu'un rivet 23 par exemple. Chaque bande 21,22 comprend, en référence aux figures 1,4,5 et 6, des moyens de fixation mâles 24 solidaires de la face externe des bandes 21,22 à proximité de l'extrémité solidaire de la coque interne, lesdits moyens de fixation mâles 24 étant aptes à coopérer avec les fines boucles de la face interne des bandes de tissu velouté 21,22. On entend par tissu velouté, un tissu à deux chaînes superposées dont l'une produit le fond du tissu et l'autre le velouté par de fines boucles sur ses faces. Par ailleurs, les boucles du tissu velouté des bandes 21,22 sont aptes à coopérer avec des moyens de fixation mâles 25 solidaires de la paroi externe de la seconde coque externe 2.

En référence aux figures 1, 2 et 4, les moyens de fixations mâles 25 solidaires de la paroi externe de la coque externe 2 de l'attelle et aptes à coopérer avec les fines boucles de la bande 21,22 sont positionnés respectivement dans deux creux 26 pratiqués sur la paroi externe de la coque externe 2 de telle sorte que les moyens de fixation 25 soient affleurant à la surface de ladite paroi externe, lesdits moyens de fixation mâles 25 étant avantageusement collés au fond des creux 26.

Par ailleurs, les moyens de fixation mâles consistent dans des crochets et les moyens de fixation femelles consistent dans des boucles, lesdits crochets étant aptes à coopérer avec les boucles et inversement pour former une fixation du type Velcro (marque déposée).

Selon une variante d'exécution particulièrement avantageuse de l'attelle suivant l'invention, en référence aux figures 2 et 9, les moyens de fixation mâles 25 sont remplacés par deux gorges transversales 27, représentées en traits mixtes sur la figure 2, de sections droites globalement rectangulaires, s'étendant perpendiculairement aux bords longitudinaux de la coque externe 2 dans la partie centrale 9 de ladite coque 2 et dont le fond 28 présente une surface rugueuse. Ainsi, les gorges 27 permettent de maintenir en position les bandes de tissu 21,22 qui ne peuvent pas glisser le long de l'axe longitudinal de la coque externe 2 et la surface rugueuse des fonds 28 desdites gorges 27 empêchent tout coulissement transversal des bandes 21,22 en coopérant avec les fines boucles desdites bandes.

Il est bien évident que la surface rugueuse peut consister dans une matière anti-dérapante ou analogue obtenue soit lors du moulage de la coque 2 soit par collage de ladite matière sur les fonds 28 des gorges 27.

En référence aux figures 1 à 4, chaque coque 1,2 comprend à son extrémité inférieure deux lumières 29 et 30 horizontales parallèles de formes globalement rectangulaires, positionnées l'une 30 au dessus de l'autre 29 et dans lesquelles peuvent être enfilées des pattes 31 et 32 en tissu s'étendant de part et d'autre de la base 5 apte à être positionné sous le talon. Chaque patte 31,32 est enfilée en passant sous une coque 1,2, en introduisant ensuite son extrémité libre dans la première lumière inférieure 29 depuis l'extérieur vers l'intérieur de la coque 1,2, puis en introduisant ladite extrémité dans la seconde lumière supérieure 30 depuis l'intérieur vers l'extérieur de ladite coque 1,2 avant de solidariser son extrémité libre sur la paroi externe de la coque 1,2 juste au dessus des lumières 29,30. Dans cet exemple particulier de réalisation, la première patte 31 est obtenue dans un tissu velouté et comprend sur sa face interne, c'est-à-dire la face venant en regard de la paroi externe des coques 1,2, des fines boucles 33 aptes à coopérer avec des moyens de fixation mâles 34 positionnés sur la paroi externe de la coque externe 2 juste au dessus des lumières 29,30. Lesdits moyens de fixation mâles 34 sont avantageusement positionnés dans un creux 35 pratiqué sur la paroi externe de la coque 2 de telle sorte que les moyens de fixation mâles 34 soient affleurants à la surface de ladite paroi externe.

L'extrémité libre de la seconde patte 32, est solidarisée à la paroi externe de la coque interne 1 par un rivet 36.

Il va de soi que la seconde patte 32 peut également comprendre sur sa face interne des fines boucles aptes à coopérer avec des moyens de fixation mâles, positionnés avantageusement dans un creux pratiqué sur la paroi externe de la coque interne juste au dessus des lumières 29,30.

Accessoirement, on observera que la partie des coques 1 et 2 s'étendant entre les lumières 29 et 30 est légèrement en retrait de telle sorte que les pattes 31 et 32 s'étendant entre lesdites lumières 29 et 30 sur la face interne des coques 1 et 2 soient affleurantes à la paroi interne desdites coques évitant ainsi toute surépaisseur qui gênerait l'utilisateur.

Par ailleurs, en référence aux figures 1 et 8, la base 5 présente une forme générale de pied et comprend sur la face supérieure de la base 5, c'est-à-dire la face de la base 5 en regard du talon, une représentation stylisée 37 d'un pied gauche de telle sorte que l'utilisateur puisse repérer facilement l'attelle qu'il doit positionner sur sa cheville gauche.

Il est bien évident que la base 5 de l'attelle correspondant à la cheville droite comprend sur sa face supérieure une représentation stylisée d'un pied droit.

Accessoirement, en référence à la figure 4, l'attelle comprend avantageusement une bande de tissu velouté dite de « strapping » 38 comprenant sur l'une de ses faces des moyens de fixation mâles 39 et 40 respectivement positionnées aux extrémités libres de ladite bande de « strapping » 38 et aptes à coopérer respectivement avec les fines boucles du tissus velouté de la face externe de la patte 34 et de la face externe de la bande 22 ou 21, la bande de « strapping » 38 étant enroulée autour des coques 1,2 depuis leurs extrémités inférieures jusqu'aux moyens 6 pour maintenir en position les coques 1,2 de part et d'autre de la cheville. On observera que l'on entend par le terme « strapping » l'application d'une bande de contention autour d'un membre du corps. Cette bande de « strapping » 38 permet de ramener vers l'intérieur le pied qui lors de la marche à tendance à pivoter vers l'extérieur en raison de la laxité des tendons de la cheville procurée par l'entorse.

Enfin, il va de soi que l'attelle pour cheville selon l'invention peut être adaptée à toutes les articulations d'un corps humain ou d'un animal, tel que le genou, le coude ou le poignet par exemple, de telles attelles ne comprenant alors qu'une seule coque, et que les exemples que l'on vient de donner ne sont que des illustrations particulières en aucun cas limitatives quant aux domaines d'application de l'invention.

## Revendications

1. - Attelle pour une articulation reliant deux membres d'un corps humain ou d'un animal, telle que la cheville, le genou ou le coude par exemple, constituée d'au moins une coque (1,2) rigide globalement concave, constituée d'un unique élément comprenant au moins une zone flexible (7,8 ;12,13), aptes à être positionnée autour de l'articulation, en appui sur ladite articulation, et comprenant sur sa face interne,c'est-à-dire sur sa face concave, une chambre (3,4) réalisée en une matière plastique souple pouvant être mise sous pression par tout moyen approprié, positionnée sur la face interne de la coque (1,2) pour fournir un coussin de support entre ladite coque (1,2) et l'articulation, et recouvrant une partie au moins de la face interne de la coque (1,2), ladite attelle comprenant des moyens (6) pour maintenir ladite coque (1,2) en position autour de l'articulation, **caractérisée en ce que** la zone flexible (7,8 ; 2, 13) est obtenue dans un co-polymère blocs styrène éthylène butylène styrène (SEBS) chimiquement lié à la partie rigide de la coque (1,2) obtenue dans du polypropylène copolymère (PPe) ou du polyamide 6 (PA 6) afin d'éviter toute blessure du tissu oedémateux apparu consécutivement à une entorse ou une foulure de ladite articulation.

2. - Attelle suivant la revendication 1 **caractérisée en ce que** la coque (1,2) est rigide dans sa partie centrale (9,14) et flexible (7,8 ; 12,13) le long de ses bords longitudinaux.

3. - Attelle suivant l'une quelconque des revendications 1 ou 2 **caractérisée en ce que** la coque (1,2) comprend dans sa partie centrale (9,14) une zone flexible (10,15) correspondant à une zone d'appui de ladite coque (1,2) sur une excroissance de l'articulation.

4. - Attelle suivant l'une quelconque des revendications précédentes **caractérisée en ce que** la ou les zones flexibles (7,8, 10,12, 13,15) consistent dans une zone de matière souple mélangée avec de la matière synthétique rigide dans laquelle est obtenue la partie rigide (9,14) de chaque coque (1,2).

5. - Attelle suivant l'une quelconque des revendications précédentes **caractérisée en ce qu'elle** comprend un élément compressible poreux (18) positionné à l'intérieur de chaque chambre (3,4) remplissant sensiblement le volume intérieur de chacune desdites chambres (3,4) lorsque ces dernières ne sont pas mises sous pression.

6. - Attelle suivant la revendication 5 **caractérisée en ce que** l'élément compressible poreux (18) consiste dans une mousse à résilience lente.

7. - Attelle suivant l'une quelconque des revendications précédentes **caractérisée en ce que** les moyens (6) pour maintenir en position la ou les coques autour de l'articulation sont constitués d'au moins deux bandes de tissu velouté (21,22), c'est-à-dire un tissu comprenant de fines boucles sur ses faces, chacune des bandes (21,22) présentant d'une part, une extrémité libre solidarisée à la face externe de la coque (1,2) ou d'une première coque, de préférence la coque interne (1) de l'attelle, c'est-à-dire la coque positionnée a l'intérieur de l'articulation, par un moyen de fixation tel qu'un rivet (23) par exemple, lesdites bandes de tissu (21,22) étant aptes à coopérer avec des moyens de fixation mâles (25) solidaires de la face externe de la coque (1,2) ou d'une seconde coque dite externe (2) et d'autre part, des moyens de fixation mâles. (24) positionnés sur la face externe de la bande (21,22) à l'extrémité solidaire de la coque (1,2) ou de la coque interne (1) et aptes à coopérer avec les fines boucles de la face interne des bandes de tissu velouté (21,22).

8. - Attelle suivant la revendication 7 **caractérisé en ce que** les moyens de fixation mâles (25) consistent dans au moins deux gorges transversales (27) de section droite globalement rectangulaires, s'étendant perpendiculairement aux bords longitudinaux de la ou les coques (1,2) dans la partie centrale rigide (9,14) de la ou des coques (1,2) et dont le fond (28) présente une surface rugueuse.

9. - Attelle suivant la revendication 7 **caractérisé en ce que** les moyens de fixation mâles (25) sont positionnés dans un creux (26) pratiqué sur la paroi externe de la coque (1,2) ou de la coque externe (2) de telle sorte que les moyens de fixation (25) soient affleurant à la surface de ladite paroi externe.

10. - Attelle suivant l'une quelconque des revendications 7 ou 9 **caractérisée en ce que** les moyens de fixation mâles (24,25) consistent dans des crochets aptes à coopérer avec les fines boucles des bandes (21,22).

11. - Attelle suivant les revendications 9 et 10 **caractérisée en ce que** les moyens de fixation mâles (25) de la face externe de la coque (1,2) ou de la coque externe (1,2) de l'attelle sont collés au fond des creux (26) pratiqués sur la paroi externe de la coque (1,2) ou de la coque externe (2).

12. - Attelle de cheville comprenant au moins deux coques (1,2) suivant l'une quelconque des revendications précédentes aptes à être positionnées de part et d'autre de la cheville.

13. - Attelle de cheville suivant la revendication 12 **caractérisée en ce que** chaque coque (1,2) comprend à son extrémité inférieure deux lumières (29,30) horizontales parallèles de formes globalement rectangulaires, positionnées l'une (30) au dessus de l'autre (29), et dans lesquelles peuvent être enfilées des pattes (31,32) en tissu velouté s'étendant de part et d'autre d'une base (5) apte à être positionnée sous le talon, chaque patte (31,32) étant enfilée en passant sous une coque (1,2), en introduisant ensuite son extrémité libre dans la première lumière inférieure (29) depuis l'extérieur vers l'intérieur de la coque (1,2), puis en introduisant ladite extrémité libre dans la seconde lumière supérieure (30) depuis l'intérieur vers l'extérieur de ladite coque (1,2) avant de solidariser son extrémité libre sur la paroi externe de la coque juste au dessus des lumières (29,30).

14. - Attelle de cheville suivant la revendication 13 **caractérisée en ce que** l'extrémité libre d'au moins une patte (31,32) comprend sur sa face interne, c'est-à-dire la face venant en regard de la paroi externe des coques (1,2), de fines boucles (33) aptes à coopérer avec des moyens de fixation mâles (34) positionnés sur la paroi externe des coques (1,2) juste au dessus des lumières (29,30).

15. - Attelle de cheville suivant la revendication 14 **caractérisée en ce que** les moyens de fixation mâles (34) sont positionnés dans un creux (35) pratiqué sur la paroi externe de la coque (1,2) de telle sorte que les moyens de fixation (34) soient affleurant à la surface de ladite paroi externe.

16. - Attelle de cheville suivant l'une quelconque des revendications 13 à 15 **caractérisée en ce que** la base (5) présente une forme générale de pied.

17. - Attelle de cheville suivant l'une quelconque des revendications 13 à 16 **caractérisée en ce qu'elle** comprend sur la face supérieure de la base (5), c'est-à-dire sur la face de la base (5) en regard du talon, une représentation stylisée (37) d'un pied droit ou gauche.

18. - Attelle de cheville suivant l'une quelconque des revendications 13 à 17 **caractérisée en ce que** l'extrémité libre de l'une des pattes (31,32) est solidarisée à la paroi externe d'une coque (1,2) par un rivet (36).

19. - Attelle de cheville suivant l'une quelconque des revendications 13 à 18 **caractérisée en ce qu'elle** comprend une bande de tissus dite.de « strapping » (38) comprenant sur l'une de ses faces des moyens de fixation mâles (39) et (40) respectivement positionnées aux extrémités libres de ladite bande de « strapping » (38) et aptes à coopérer respectivement avec les fines boucles du tissus velouté de la face externe de la patte (34) et de la face externe de la bande (22) ou (21), la bande de « strapping » (38) étant enroulée autour des coques (1,2) depuis leurs extrémités inférieures jusqu'aux moyens (6) pour maintenir en position les coques 1,2 de part et d'autre de la cheville.

20. - Procédé de fabrication des coques (1,2) d'une attelle pour une articulation reliant deux membres du corps humain, telle que la cheville, le genou ou le coude par exemple, constituée d'au moins deux coques (1,2) rigides globalement concaves, aptes à être positionnées de part et d'autre de l'articulation, en appui sur ladite articulation, suivant l'une quelconque des revendications 1 à 6 **caractérisé en ce qu'il** consiste à introduire une matière synthétique en polypropylène co-polymère (PPe) ou en polyamide 6 (PA 6) liquide à chaud qui se rigidifie en refroidissant dans un moule de forme correspondent à la forme de la coque (1,2) à obtenir, puis à introduire dans ledit polypropylène co-polymère (PPe) ou ledit polyamide 6 (PA6) un co-polymère blocs styrène éthylène butylène styrène (SEBS) dans au moins une zone du moule.

## Claims

1. Splint for a joint connecting two parts of a human or animal body, such as the ankle, the knee or the elbow, for example, consisting of at least one rigid, generally concave shell (1, 2) consisting of a single element including at least one flexible area (7, 8; 12, 13), capable of being positioned around the joint, in contact with said joint, and including, on its internal face, i.e. on its concave face, a chamber (3, 4) made of a flexible plastic material capable of being subjected to pressure by any suitable means, positioned on the internal face of the shell (1, 2) so as to provide a support cushion between said shell (1, 2) and the joint, and covering at least a portion of the internal face of the shell (1, 2), which splint includes means (6) for holding said shell (1, 2) in position around the joint, **characterised in that** the flexible area (7, 8; 2, 13) is made of a styrene-ethylene-butylene-styrene (SEBS) block copolymer chemically bonded to the rigid portion of the shell (1, 2) made of propylene copolymer (PPe) or polyamide 6 (PA 6), so as to prevent any injury to the oedematous tissue resulting from a sprain or a strain of said joint.

2. Splint according to claim 1, **characterised in that** the shell (1, 2) is rigid in its central portion (9, 14) and flexible (7, 8; 12, 13) along its longitudinal edges.

3. Splint according to either one of claims 1 or 2, **characterised in that** the shell (1, 2) includes, in its central portion (9, 14), a flexible area (10, 15) corresponding to an area of contact of said shell (1, 2) on a protuberance of the joint.

4. Splint according to any one of the previous claims, **characterised in that** the flexible area(s) (7, 8, 10, 12, 13, 15) consist of an area made of a flexible material mixed with a rigid synthetic material of which the rigid portion (9, 14) of each shell (1, 2) is made.

5. Splint according to any one of the previous claims, **characterised in that** it includes a porous compressible element (18) positioned inside each chamber (3, 4) substantially filling the interior volume of each of said chambers (3, 4) when the latter are not subjected to pressure.

6. Splint according to claim 5, **characterised in that** the porous compressible element (18) consists of a slow resilience foam.

7. Splint according to any one of the previous claims, **characterised in that** the means (6) for holding the shell(s) in position around the joint consist of at least two velvety fabric strips (21, 22), i.e. a fabric including fine loops on its faces, in which each of the strips (21, 22) has a free end secured to the external face of the shell (1, 2) or a first shell, preferably the internal shell (1) of the splint, i.e. the shell positioned inside the joint, by attachment means such as a rivet (23), for example, which fabric strips (21, 22) are capable of cooperating with male attachment means (25) secured to the external face of the shell (1, 2) or a second so-called external shell (2), and male attachment means (24) positioned on the external face of the band (21, 22) at the end secured to the shell (1, 2) or the internal shell (1) and capable of cooperating with the fine loops of the internal face of the velvety fabric strips (21, 22).

8. Splint according to claim 7, **characterised in that** the male attachment means (25) consist of at least two transverse grooves (27) with a generally rectangular cross-section, extending perpendicularly to the longitudinal edges of the shell(s) (1, 2) in the rigid central portion (9, 14) of the shell(s) (1, 2) and of which the base (28) has a rough surface.

9. Splint according to claim 7, **characterised in that** the male attachment means (25) are positioned in a recess (26) formed on an external wall of the shell (1, 2) or the external shell (2) so that the attachment means (25) are flush with the surface of said external wall.

10. Splint according to any one of claims 7 to 9, **characterised in that** the male attachment means (24, 25) consist of hooks capable of cooperating with the fine loops of the strips (21, 22).

11. Splint according to claims 9 and 10, **characterised in that** the male attachment means (25) of the external face of the shell (1, 2) or the external shell (1, 2) of the splint are adhered to the base of the recesses (26) formed on the external wall of the shell (1, 2) or the external shell (2).

12. Ankle splint including at least two shells (1, 2) according to any one of the previous claims, capable of being positioned on each side of the ankle.

13. Ankle splint according to claim 12, **characterised in that** each shell (1, 2) includes, at its lower end, two parallel horizontal holes (29, 30) with a generally rectangular shape, positioned one (30) on top of the other (29), and in which straps (31, 32) can be threaded, made of a velvety fabric extending on each side of a base (5) capable of being positioned under the ankle, wherein each strap (31, 32) is threaded by passing under a shell (1, 2), then inserting its free end into the first lower hole (29) from the outside to the inside of the shell (1, 2), then by inserting said free end into the second upper hole (30) from the inside to the outside of said shell (1, 2) before securing its free end to the external wall of the shell just above the holes (29, 30).

14. Ankle splint according to claim 13, **characterised in that** the free end of at least one strap (31, 32) includes, on its internal face, i.e. the face opposite the external wall of the shells (1, 2), fine loops (33) capable of cooperating with male attachment means (34) positioned on the external wall of the shells (1, 2) just above the holes (29, 30).

15. Ankle splint according to claim 14, **characterised in that** the male attachment means (34) are positioned in a recess (35) formed on the external wall of the shell (1, 2) so that the attachment means (34) are flush with the surface of said external wall.

16. Ankle splint according to any one of claims 13 to 15, **characterised in that** the base (5) has a general foot shape.

17. Ankle splint according to any one of claims 13 to 16, **characterised in that** it includes, on the upper face of the base (5), i.e. on the face of the base (5) opposite the heel, a stylised representation (37) of a right or left foot.

18. Ankle splint according to any one of claims 13 to 17, **characterised in that** the free end of one of the straps (31, 32) is secured to the external wall of a shell (1, 2) by a rivet (36).

19. Ankle splint according to any one of claims 13 to 18, **characterised in that** it includes a strip of so-called "strapping" fabric (38) including, on one of its faces, male attachment means (39) and (4), respectively, positioned at the free ends of said "strapping" strip (38) and capable of cooperating respectively with the fine loops of the velvety fabric of the external face of the strap (34) and the external face of the strip (22) or (21), in which the "strapping" strip (38) is wound around the shells (1, 2) from their lower ends to the means (6) for holding the shells (1, 2) in position on each side of the ankle.

20. Method for producing shells (1, 2) of a splint for a joint connecting two parts of the human body, such as the ankle, the knee or the elbow, for example, consisting of at least two generally concave rigid shells (1, 2), capable of being positioned on each side of the joint, in contact with said joint, according to any one of claims 1 to 6, **characterised in that** it consists of inserting a hot, liquid synthetic propylene copolymer (PPe) or polyamide 6 (PA 6) material that solidifies when it cools in a mould of a shape corresponding to the shape of the shell (1, 2) to be obtained, then adding, to said propylene copolymer (PPe) or said polyamide 6 (PA 6), a styrene-ethylene-butylene-styrene (SEBS) block copolymer to at least one area of the mould.

## Patentansprüche

1. Schiene für ein Gelenk, das zwei Gliedmaßen eines menschlichen Körpers oder eines Tieres verbindet, wie beispielsweise das Sprunggelenk, das Knie oder der Ellenbogen, die aus wenigstens einer im wesentlichen konkaven steifen Schale (1, 2) gebildet ist, die aus einem einzigen Element gebildet ist, das wenigstens eine biegsame Zone (7, 8; 12, 13) umfasst, die dafür geeignet ist, um das Gelenk herum und auf das Gelenk abstützend positioniert zu werden, wobei sie auf ihrer Innenseite, das heißt auf ihrer konkaven Seite, eine Kammer (3, 4) umfasst, die aus einem weichen Kunststoffmaterial hergestellt ist und die durch jedes geeignete Mittel unter Druck gesetzt werden kann, wobei sie auf der Innenseite der Schale (1, 2) positioniert ist, um ein Stützkissen zwischen der Schale (1, 2) und dem Gelenk zu bilden, wobei sie wenigstens einen Teil der Innenseite der Schale (1, 2) abdeckt, wobei die Schiene Mittel (6) umfasst, um die Schale (1, 2) um das Gelenk herum in Position zu halten, **dadurch gekennzeichnet, dass** die biegsame Zone (7, 8; 12, 13) aus einem Styrol-Ethylen-Butylen-Styrol Blockcopolymer (SEBS) realisiert ist, das chemisch mit dem steifen Teil der Schale (1, 2) verbunden ist, der aus einem Polypropylen-Copolymer (PPe) oder Polyamid 6 (PA 6) realisiert ist, um jede Verletzung des ödematöses Gewebes zu vermeiden, das als Folge einer Verstauchung oder einer Verletzung des Gelenks aufgetreten ist.

2. Schiene nach Anspruch 1, **dadurch gekennzeichnet, dass** die Schale (1, 2) in ihrem zentralen Teil (9, 14) steif und entlang ihrer in Längsrichtung verlaufenden Ränder biegsam (7, 8; 12, 13) ist.

3. Schiene nach einem der Ansprüche 1 oder 2, **dadurch gekennzeichnet, dass** die Schale (1, 2) in ihrem zentralen Teil (9, 14) eine biegsame Zone (10, 15) umfasst, die einer Zone zur Abstützung der Schale (1, 2) auf einem Vorsprung des Gelenks entspricht.

4. Schiene nach einem der vorhergehenden Ansprüche, **dadurch gekennzeichnet, dass** die eine oder mehreren biegsamen Zonen (7,8, 10,12, 13,15) aus einer Zone weichen Materials gemischt mit steifem Kunststoffmaterial, aus dem der steife Teil (9, 14) jeder Schale (1, 2) realisiert ist, bestehen.

5. Schiene nach einem der vorhergehenden Ansprüche, **dadurch gekennzeichnet, dass** sie ein poröses zusammendrückbares Element (18) umfasst, das im Inneren jeder Kammer (3, 4) angeordnet ist und das das innere Volumen jeder der Kammern (3, 4) im wesentlichen ausfüllt, wenn letztere keinem Druck ausgesetzt sind.

6. Schiene nach Anspruch 5, **dadurch gekennzeichnet, dass** das poröse zusammendrückbare Element (18) aus einem Schaum mit langsamer Rückstellelastizität besteht.

7. Schiene nach einem der vorhergehenden Ansprüche, **dadurch gekennzeichnet, dass** die Mittel (6), um die einen oder mehreren Schalen um das Gelenk herum in Position zu halten, aus wenigstens zwei Bändern aus veloursartigem Gewebe (21, 22) bestehen, das heißt einem Gewebe, das auf seinen Flächen feine Schlingen umfasst, wobei jedes der Bänder (21, 22) zum einen ein freies Ende hat, das fest an der Außenseite der Schale (1, 2) oder einer ersten Schale, wobei dies vorzugsweise die innere Schale (1) der Schiene ist, das heißt die Schale, die auf der Innenseite des Gelenks sitzt, angebracht ist, und dies beispielsweise durch ein Befestigungsmittel wie etwa einem Niet (23), wobei die Gewebebänder (21, 22) dafür geeignet sind, mit männlichen Befestigungsmitteln (25) zusammenzuarbeiten, die fest auf der Außenseite der Schale (1, 2) oder einer zweiten Schale, die als äußere Schale (2) bezeichnet wird, befestigt sind, und zum anderen männliche Befestigungsmittel (24) hat, die auf der Außenseite des Bands (21, 22) an dem Ende, das fest an der Schale (1, 2) oder der inneren Schale (1) angebracht ist, positioniert sind und die dafür geeignet sind, mit den feinen Schlingen der Innenseite der Bänder aus veloursartigem Gewebe (21, 22) zusammenzuarbeiten.

8. Schiene nach Anspruch 7, **dadurch gekennzeichnet, dass** die männlichen Befestigungsmittel (25) aus wenigstens zwei quer verlaufenden Rillen (27) mit im wesentlichen rechteckigem Querschnitt bestehen, die sich senkrecht zu den in Längsrichtung verlaufenden Rändern der einen oder mehreren Schalen (1, 2) im steifen zentralen Teil (9, 14) der einen oder mehreren Schalen (1, 2) erstrecken und deren Boden (28) eine raue Oberfläche hat.

9. Schiene nach Anspruch 7, **dadurch gekennzeichnet, dass** die männlichen Befestigungsmittel (25) in einer Aushöhlung (26) angeordnet sind, die auf der Außenwand der Schale (1, 2) oder der äußeren Schale (2) derart ausgeführt ist, dass die Befestigungsmittel (25) in der Oberfläche der Außenwand bündig eingelassen sind.

10. Schiene nach einem der Ansprüche 7 oder 9, **dadurch gekennzeichnet, dass** die männlichen Befestigungsmittel (24, 25) aus Haken bestehen, die dafür geeignet sind, mit den feinen Schlingen der Bänder (21, 22) zusammenzuarbeiten.

11. Schiene nach den Ansprüchen 9 und 10, **dadurch gekennzeichnet, dass** die männlichen Befestigungsmittel (24, 25) der Außenseite der Schale (1, 2) oder der äußeren Schale (1, 2) der Schiene in den Boden der Aushöhlungen (26) geklebt sind, die an der Außenwand der Schale (1, 2) oder der äußeren Schale (2) ausgeführt sind.

12. Sprunggelenkschiene, die wenigstens zwei Schalen (1, 2) nach einem der vorhergehenden Ansprüche umfasst, die dafür geeignet sind, auf beiden Seiten des Sprunggelenks angeordnet zu werden.

13. Sprunggelenkschiene nach Anspruch 12, **dadurch gekennzeichnet, dass** jede Schale (1, 2) an ihrem unteren Ende zwei horizontale parallele Öffnungen (29, 30) mit im wesentlichen rechteckigen Formen umfasst, wobei die eine (30) über der anderen (29) angeordnet ist und in die Lappen (31, 32) aus veloursartigem Gewebe eingefädelt werden können, die sich auf beiden Seiten eines Basisteils (5) erstrecken, das dafür geeignet ist, unter der Ferse angeordnet zu werden, wobei jeder Lappen (31, 32) eingefädelt wird, indem er unter einer Schale (1, 2) durchläuft, anschließend sein freies Ende in die erste untere Öffnung (29) von der Außenseite zur Innenseite der Schale (1, 2) eingeführt wird, danach das freie Ende in die zweite obere Öffnung (30) von der Innenseite zur Außenseite der Schale (1, 2) eingeführt wird, bevor sein freies Ende auf der Außenwand der Schale gerade oberhalb der Öffnungen (29, 30) befestigt wird.

14. Sprunggelenkschiene nach Anspruch 13, **dadurch gekennzeichnet, dass** das freie Ende wenigstens eines Lappens (31, 32) auf seiner Innenseite, das heißt der Seite, die gegenüber der Außenwand der Schalen (1, 2) zu liegen kommt, feine Schlingen (33) umfasst, die dafür geeignet sind, mit männlichen Befestigungsmitteln (34) zusammenzuarbeiten, die auf der Außenwand der Schalen (1, 2) gerade oberhalb der Öffnungen (29, 30) angeordnet sind.

15. Sprunggelenkschiene nach Anspruch 14, **dadurch gekennzeichnet, dass** die männlichen Befestigungsmittel (34) in einer Aushöhlung (35) angeordnet sind, die an der Außenwand der Schale (1, 2) derart ausgeführt ist, dass die Befestigungsmittel (34) in der Oberfläche der Außenwand bündig eingelassen sind.

16. Sprunggelenkschiene nach einem der Ansprüche 13 bis 15, **dadurch gekennzeichnet, dass** das Basisteil (5) allgemein fußförmig ist.

17. Sprunggelenkschiene nach einem der Ansprüche 13 bis 16, **dadurch gekennzeichnet, dass** sie auf der Oberseite des Basisteils (5), das heißt auf der Seite des Basisteils (5), das der Ferse gegenübersteht, eine stilisierte Darstellung (37) eines rechten oder linken Fußes umfasst.

18. Sprunggelenkschiene nach einem der Ansprüche 13 bis 17, **dadurch gekennzeichnet, dass** das freie Ende eines der Lappen (31, 32) an der Außenwand einer der Schalen (1, 2) durch einen Niet (36) befestigt ist.

19. Sprunggelenkschiene nach einem der Ansprüche 13 bis 18, **dadurch gekennzeichnet, dass** sie ein Gewebeband umfasst, das als "Festschnall"-Band (38) bezeichnet wird und das auf einer seiner Flächen männliche Befestigungsmittel (39) und (40) umfasst, die jeweils an den freien Enden des "Festschnall"-Bands (38) angeordnet sind und die dafür eingerichtet sind, jeweils mit den feinen Schlingen des veloursartigen Gewebes der Außenseite der Lappen (34) und der Außenseite des Bands (22) oder (21) zusammenzuarbeiten, wobei das "Festschnall"-Band (38) um die Schalen (1, 2) gewickelt wird, und zwar von deren unteren Enden bis zu den Mitteln (6) um die Schalen (1, 2) auf beiden Seiten des Sprunggelenks in Position zu halten.

20. Herstellungsverfahren für Schalen (1, 2) einer Schiene für ein Gelenk, das zwei Gliedmaßen des menschlichen Körpers verbindet, wie beispielsweise dem Sprunggelenk, dem Knie oder dem Ellenbogen, die aus wenigstens zwei im wesentlichen konkaven steifen Schalen (1, 2) gebildet wird, die dafür geeignet sind, auf beiden Seiten des Gelenks und auf das Gelenk abstützend positioniert zu werden, nach einem der Ansprüche 1 bis 6, **dadurch gekennzeichnet, dass** es darin besteht, ein Kunststoffmaterial aus flüssigem Polypropylen-Copolymer (PPe) oder Polyamid 6 (PA 6) heiß einzuführen, das sich abkühlend in einer Form mit einer Formung, die der zu erhaltenden Schale (1, 2) entspricht, verfestigt, und danach in das Polypropylen-Copolymer (PPe) oder das Polyamid 6 (PA 6) in wenigstens einer Zone der Form ein Styrol-Ethylen-Butylen-Styrol Blockcopolymer (SEBS) einzuführen.
